# EUROPEAN PATENT SPECIFICATION

(11) **EP 2 527 361 B1**
(45) Date of publication and mention of the grant of the patent: **16.11.2016**
(21) Application number: 11305651.9
(22) Date of filing: 27.05.2011
(51) Int. Cl.: C07K 14/16, G01N 33/68, C07K 16/10, C12N 5/16

(54) **Biomarkers for in vitro prognosis and diagnosis of graft and transplant rejection**
Biomarker für In-vitro-Prognose und -Diagnose der Transplantatabstoßung
Biomarqueurs pour le pronostic in vitro et le diagnostic du rejet de greffes et de transplantations

(43) Date of publication of application: 28.11.2012
(73) Proprietor: Biovialife Inc., Toronto ON M5A 3W7 (CA)
(72) Inventor: Agbalika, Felix, 75020 Paris (FR)
(74) Representative: Grosset-Fournier, Chantal Catherine

(56) References cited:
- WO-A1-2009/089568
- WO-A1-2010/083121
- RONG CHEN ET AL: "Differentially Expressed RNA from Public Microarray Data Identifies Serum Protein Biomarkers for Cross-Organ Transplant Rejection and Other Conditions", PLOS COMPUTATIONAL BIOLOGY, vol. 6, no. 9, 1 January 2010 (2010-01-01) , page E1000940, XP55010324, ISSN: 1553-734X, DOI: 10.1371/journal.pcbi.1000940

## Description

The present invention relates to novel peptides and compositions containing the same for their use as a tool in prognosis or diagnosis of a grafted organ distress, notably of graft or transplant rejection.

The main function of kidney in living organisms is to clear the blood of waste products or toxic substances. Kidney injury may damage renal structure (glomerular or tubule cell deletion, fibrosis, and renal vasculature alterations) leading to chronic renal excretory dysfunction **(**Lopez Novoa et al., J Transl Med 2011, 9: 13). Evolution of chronic kidney diseases can be monitored by key pathological events such as percentage of nephron functionally active, glomerular filtration rate, renal excretory function or by measurement of plasma and urine biomarkers (creatinine, uric acid, ...). Progression of chronic kidney diseases has been classified in five stages according to renal dysfunction and renal damage (Levey et al., Ann Intern Med 2003, 139, 137-147)*.* Stage 1 is generally a silent phase in which renal dysfunction can be reversed whereas late stage 4 and stage 5 correspond to renal failure and need for renal replacement therapy such as dialysis or kidney transplant whenever feasible.

Organ transplant can also occur in other organ systems such as circulatory system (heart), digestive system (liver) and respiratory system (lung). Patients suffering from leukaemia can also receive soft tissue graft, i.e bone marrow graft.

Each year in Europe, more than 15 000 renal grafts, 5 000 liver grafts, 2 000 heart transplants and 1 000 lung grafts are performed. Organ transplantation is often the only treatment for end state organ failure. Cells and tissues, such as haematopoietic stem cells or heart valve are also used for transplantation to restore essential functions. Adverse events and reactions such as graft or transplant rejection often occurred in recipients. This could lead at least to the damage of the graft and eventually to its loss. The use of immunosuppressive drugs helps to improve results of transplantation by modulating immune reaction of recipients. However, these drugs induce a set of adverse events for patients that limit their quality of life or survival expectancies. The most frequent side-effects are nephrotoxicity, cancer and cardiovascular events.

Molecular mechanisms underlying graft or transplant rejection have been identified. Immune rejection is mediated by activation of recipient T-cells against donor cells. Recipient T-cells recognize donor's major histocompatibility complex (MHC) class-1 encoded antigens. Infiltration of the transplanted organ by T-cells and other mononuclear leukocytes (such as B-cell and natural killer cells) is responsible for damaging the graft, by activating the complement system and mononuclear cells. Both CD4+ and CD8+ T-cells participate in acute cellular rejection. The production of anti-donor MHC class I and class II antibodies is also associated with acute and chronic graft damage. In chronic graft rejection, the recipient immune response activation is probably not the only cause of rejection. Additional causes such as fibrosis, viral infection or recurrence of the original disease might play an important role in graft rejection **(**Sanchez-Fueyo et al., Gastroenterology 2011, 140, 51-64)*.*

Rejection episodes are diagnosed by conventional clinical parameters, which are specific of the grafted organ and dependant of genetic factors, and are mostly confirmed by biopsy. At the present time, tissue allograft biopsy with conventional histological examination remains the gold standard for diagnosing rejection among transplanted patients **(**Racusen et al., The Banff 97 working classification of renal allograft pathology, Kidney International 1999, 55, 713-723*).* As protocol biopsies are invasive methods, physicians' intervention is needed to collect a small piece of tissue of the grafted organ. Though these tests quite accurately predict graft failure, they usually detect it at a relatively late stage when extensive tissue damage has already occurred. Early detection of graft failure before tissue damage occurrence may help physicians monitoring immunosuppressive drugs more efficiently, minimizing their side effects while prolonging graft survival rates. Identification of proteins in sera indicating which patients are at highest risk of subsequent acute allograft rejection and chronic allograft dysfunction may be helpful in developing new *in vitro* prognosis and diagnosis assays applicable to a body fluid sample.

The identification of biomarkers for graft and transplant rejection remains challenging. In the past ten years, different strategies have been developed to identify such markers. The first strategy consists in selecting a specific protein for its known implication in the immune reaction system. US 2008/274910 discloses that enhanced expression profile of TIRC-7 (T-cell immune response cDNA 7) in renal grafted patients' blood is determinative for early activation of immune activation which could lead to graft rejection.

More ubiquitous proteins (not involved in immune system) such as TRIB-1, a MAP kinase activator (WO 20071138011), or lysozyme (JP 2006/6177679) have been identified as markers for *in vitro* diagnosis and or prognosis of respectively renal or enteric acute graft rejection. In all the previous examples, a single protein was used.

Another strategy was to study the transcriptional profile of a transplanted organ. In WO 2004/074815, the inventors studied expression of known genes that encodes pro-inflammatory or adhesion proteins that mediate immune activation and anti-apoptotic proteins on transplanted renal tissue within 15 minutes after vascular reperfusion. The results demonstrate that delayed graft function, acute rejection or delayed rejection can be predicted from the differential expression of clusters of genes in combination with clinical information.

A more global approach was recently developed in the field of graft rejection. Genomic or proteomic technologies have enabled gene or protein expression profiling of many human diseases.

Microarray tools offer the possibility to detect patterns of differentially expressed gene among hundreds or thousands of genes. In WO 2010/083121, the inventors screened on microarrays platforms the level of expression of whole genome on blood sample of a patient who has received a kidney graft and identify a set of genes which level of expression was confirmed by RT-PCR technology. The gene expression profile is employed to predict the occurrence of an acute rejection response between 6 to 3 months in advance. A similar strategy was developed by *using in silico* data from biopsy-based gene expression microarray studies from renal and cardiac transplantation **(**Chen et al., PLoS Computational Biology 2010, 6(9), 1-12*).* This led to the identification of the three cross-organ acute rejection protein biomarkers i.e, PECAM-1, CXCL-9 and CD44.

In heart transplantation, the Cardiac Allograft Rejection Gene Expression Observation (CARGO) studies led to the development of non-invasive, FDA homologated, commercially available test for acute rejection (*www. allomap. com)*. Measured expression levels of 20 genes are transformed by an algorithm into an integer value that is reported to a score. The clinician uses this score in the overall assessment of the probability of rejection at the time of the testing. This *in vitro* diagnosis assay can be performed between 2 months to over 6 months post transplanting to assess rejection.

Measurement of protein plasma concentration by using iTRAQ-MALDI-TOF/TOF methodology identified 18 protein group codes with differential relative concentration (Cohen Freue et al., Molecular & Cellular Proteomics 9.9 2010, 1954-1967)*.* Some of these proteins have been previously associated with acute renal graft rejection such as those involved in complement system (MBL2-Mannose Binding protein C), coagulation cascade (SERPINA10 and Factor 11) or inflammatory response (Macrophage-stimulating protein-MSPT-1 and MSPT-9). In this publication, the authors also disclosed an anti-hypertrophic protein secreted by cardiomyocytes called PI16, and a vitamin E-binding member of the albumin family protein called AFM, said proteins having unknown role in renal function. This study strengthens the idea that it is possible to identify biomarkers universally present across all transplanted organs.

Identification of new proteins with unknown role in acute or chronic rejection may provide not only new assays for the prognosis and diagnosis of organ rejection but also new targets for therapeutic intervention. Hence, this could lead to total or partial withdrawal of immunosuppressive drugs or to adapt the regimen or to change the active molecule used to allow graft tolerance in recipient.

Although above mentioned genomic and proteomic technologies constitute powerful tools leading to the identification of new genes or proteins as marker of acute or chronic graft rejection, their implementation remains costly and they are not always available in hospital. Accordingly, there is still a need for alternative markers enabling simple, quick and easy to implement assays for prognosis and diagnosis of graft and transplant rejection.

Thus, one of the aims of the present invention is to provide new proteins being a specific biomarker of a grafted organ distress, notably of graft and transplant rejection.

The present invention allows not only the diagnosis but also the prognosis of graft or transplant rejection in only a few minutes.

The present invention relates to a peptide comprising or consisting of the amino acid sequence SEQ ID NO: 1. The present disclosure also describes peptide derived from SEQ ID NO: 1, said peptide having at least 65% identity with amino acids 1 to 11 of SEQ ID NO: 1 with the proviso that the peptide is not a native viral protein.

By "native viral protein" is meant a proteic sequence which comprises, corresponds to, is present in or is otherwise derived from any proteic sequence which may be found in the genome of a virus. As example of native viral protein, the gag protein from HIV is to be cited.

In a preferred embodiment, the peptide according to the invention has at least 70%, more preferably at least 75%, more preferably at least 80%, more preferably at least 85%, more preferably at least 90% and most preferably at least 95% identity with amino acids 1 to 11 of SEQ ID NO: 1.

By the expression "peptide" is meant a contiguous linear amino acid chain. This contiguous amino acid chain can be either from natural origin or artificial (from chemical synthesis). Methods of chemical synthesis of peptides are well known to one skilled in the art.

Fragments of the peptide of the amino acid sequence SEQ ID NO: 1 are also part of the present disclosure. By fragment of peptide is meant at least 5 contiguous amino acid chain selected among the eleven amino acids of peptide of SEQ ID NO: 1 which are able to bind to antibodies raised against peptide of SEQ ID NO: 1.

Variants of the peptide of the amino acid sequence SEQ ID NO: 1 which may be obtained by sequence alterations of said peptide are also part of the present disclosure. These alterations can include substitutions, deletions, insertions or mutations. Modified or unusual amino acids can also be enclosed in such variants. Functional groups present on the lateral chains of the amino acid moieties on terminal N or C groups can also be modified according to known methods in the art. Such derivatives include for example esters, aliphatic amides of the carboxyl groups and N-acyl derivates of the free aminogroups. Additionally, amino acids may be subjected to post-translational modifications such as glycosylation, sialylation, acetylation, methylation, amidation, sulfatation, formylation or phosphorylation.

Peptides and variants of the peptide comprising or consisting of the amino acid sequence SEQ ID NO: 1 which may be salted peptides are also part of the present disclosure. The salt of the carboxyl group comprises inorganic salts as for example sodium, potassium, calcium salts with organic bases as triethanolamine, arginine or lysine. The salt of the amino group comprises for example salts with inorganic acids as hydrochloric acid or with organic acids as acetic acid.

The peptide of SEQ ID NO: 1 has been called VEA195.

The peptide comprising or consisting of the amino acid sequence SEQ ID NO: 1 is derived from a sera protein of molecular weight of 25-30 kDa, isolated from grafted or transplanted host.

The present invention also relates to an isolated nucleic acid molecule encoding a peptide as defined above, in particular a nucleic acid sequence capable of encoding a peptide comprising or consisting of the amino acid sequence SEQ ID NO: 1, wherein said nucleic acid is SEQ ID NO: 2 with the proviso that the nucleic acid molecule is not a native viral nucleic acid.

The present disclosure also describes an isolated nucleic acid molecule encoding a peptide as defined above, in particular a nucleic acid sequence capable of encoding a peptide comprising or consisting of peptide derived from SEQ ID NO: 1 with the proviso that the peptide is not a native viral peptide, wherein said nucleic acid is a fragment of SEQ ID NO: 2, with the proviso that the nucleic acid molecule is not a native viral nucleic acid.

By "native viral nucleic acid" is meant a nucleic acid sequence which comprises, corresponds to, is present in or is otherwise derived from any nucleic acid sequence which may be found in the genome of a virus. As example of native nucleic acid sequence, the *gag* gene from HIV is to be cited.

Due to the degeneracy of the genetic code, the codons encoding amino acids of SEQ ID NO: 1 may differ in any of their three positions. For example the amino acid lysine can be specified by AAA or AAG codons (difference in the third position).

By the term "nucleic acid molecule" is meant a single or a double stranded DNA molecule, or a single or double stranded RNA molecule, said RNA molecule being directly deducted from DNA molecule, or a hybrid DNA/RNA molecule, which encodes for the peptide of the present invention.

The present invention also relates to a recombinant nucleic acid construct comprising the above-mentioned nucleic acid molecule operably linked to an expression vector.

By the expression "operably linked" is meant that the aforesaid nucleic acid molecule is linked in a covalent way to an expression vector, permitting ribosome to translate the nucleic acid molecule, or permitting RNA polymerase to produce a mRNA encoding the peptide according to the invention.

By the term "expression vector" is meant a molecule made of nucleic acids, in particular a plasmid, which comprises a promoter region and optionally an enhancer region. The expression vector can include other genes such as an antibiotic resistance gene, in order to select host cells that contain the nucleic acid molecule encoding the peptide according to the invention. Recombinant production techniques that use the aforementioned expression vector are well known to one skilled in the art.

The present invention also relates to a host cell comprising the above-mentioned recombinant nucleic acid construct.

By the term "host cell" is meant any living cell, in particular eukaryotic, but also bacterial cell, or any non-living medium comprising transcriptional and/or translational machinery and optionally organelles from a cell, permitting to amplify the aforesaid nucleic acid construct and/or produce the peptide according to the invention.

The present invention also relates to an antibody or fragment thereof that binds specifically to an epitope, said epitope comprising or consisting of the sequence SEQ ID NO: 1. The present disclosure also describes an antibody or fragment thereof that binds specifically to an epitope, said epitope comprising or consisting of fragments of the peptide of sequence SEQ ID NO: 1.

By the term "antibody" is meant antibody belonging to any species such as human, mouse, rat, rabbit or goat species. Antibody can also be chimeric antibody, i.e. an antibody which comprises parts originating from different species. Antibody can also be humanised. Antibody can also be antibody fragment which comprise at least one paratope such as Fab, F(ab')2 or scFv fragments. The antibody of the invention can be a polyclonal or a monoclonal antibody. Monoclonal antibody can also be an anti-idiotypic monoclonal antibody or a Fab fragment of anti-idiotypic antibody. The antibody of the invention can be a natural antibody or a recombinant antibody.

By the term "specifically" is meant that peptide interacts with one antibody without interacting substantially with another antibody which does not structurally resemble the aforesaid antibody.

Methods to generate polyclonal or monoclonal antibodies are well known by the one skilled in the art. Typically, peptide of SEQ ID NO: 1 may be injected with high molecular weight carrier protein to non human mammals (mouse, rabbit, goat or sheep for example) in order to elicit a good immune response. Immunization protocol may be performed several times before the animal sacrifice. Serum of animal is then purified on chromatography column to isolate monoclonal or polyclonal antibody of interest.

Typically, monoclonal antibody results from the selection of a hybridoma secreting an antibody raised against peptide of SEQ ID NO: 1. Such a hybridoma results from the fusion between splenic cells of non human mammal immunized with peptide of SEQ ID NO: 1 and myeloma cells of non human mammal. Selection of hybridomas occurs on the bases of their capacity to secrete antibodies able to bind to peptide of SEQ ID NO: 1.

In a preferred embodiment, the antibodies targeting the amino acid sequence SEQ ID NO: 1 are secreted by the hybridomas deposited at the CNCM (Collection Nationale de Culture de Microorganismes, Institut Pasteur, 25 rue du Docteur Roux, 75724 Paris Cedex 15, France) under the Budapest Treaty on May 4, 2011, under reference numbers CNCMI-4479, CNCM I-4480 and CNCM I-4481 which are part of the present invention.

The present disclosure also describes that the antibodies targeting a peptide derived from SEQ ID NO: 1 are secreted by the hybridomas deposited at the CNCM (Collection Nationale de Culture de Microorganismes, Institut Pasteur, 25 rue du Docteur Roux, 75724 Paris Cedex 15, France) under the Budapest Treaty on May 4, 2011, under reference numbers CNCM I-4479, CNCM I-4480 and CNCM 1-4481.

The present invention also relates to hybridomas 2C7D10E5, 4H7A7G9 and 1H8C9C10C7 deposited at the CNCM on May 4, 2011 under reference numbers respectively CNCM I-4479, CNCM I-4480 and CNCM 1-4481.

The present invention also relates to hybridomas deposited at the CNCM (Paris) under the Budapest Treaty on May 4, 2011, under reference numbers CNCM I-4479, CNCM I-4480 or CNCM I-4481, that secretes an antibody or fragment thereof that binds specifically to an epitope consisting of the peptide consisting of the amino acid sequence SEQ ID NO: 1.

The peptide according to the present invention is immunogenic, i.e. VEA195 peptide is able to induce a humoral immune response once injected in a mammal. The exact function in graft rejection of the whole protein from which VEA195 peptide has been identified is not yet known. As no one can predict whether this protein has beneficial or deleterious effect on graft rejection, two therapeutic strategies might be developed to avoid graft rejection. If the presence of that protein is needed, the administering to the recipient one or more dose of DNA vaccine before or at the same time performing engraftment with a plasmid comprising a polynucleotide encoding the recipient protein from which VEA195 peptide derived might be helpful. This approach was tested in WO 2009/114735 by using DNA vaccine consisting of a plasmid comprising a polynucleotide encoding the Bax pro-apoptotic protein. In the opposite, if the absence of that protein is needed, depletion of whole protein from which VEA195 peptide derived might be developed by using immobilization antibodies raised specifically against said VEA195 peptide. One of ordinary skills in the art might use a procedure in which blood is taken from the patient's circulation to have a depletion process applied to it before it is returned to the circulation. For example, antibodies raised against VEA195 peptide could be bound to a resin column of an apparatus circulating the blood outside the body. The 25-30 kDa protein from which VEA195 peptide has been identified would be retained on the column whereas whole blood is returned to the body of the grafted patient. The rapid depletion of this protein from blood circulation might limit graft or transplant rejection.

The present invention also relates to an antibody or fragment thereof that binds specifically to an epitope consisting of the peptide consisting of the amino acid sequence SEQ ID NO: 1 and that is secreted by one of the hybridomas of reference numbers CNCM I-4479, CNCM I-4480 and CNCM I-448, as defined above.

Another aim of the invention is to provide a composition comprising at least one peptide or one antibody as defined above.

The present invention also relates to a composition comprising a peptide consisting of the amino acid sequence SEQ ID NO: 1, or an antibody that binds specifically to an epitope consisting of the peptide consisting of the amino acid sequence SEQ ID NO: 1 or that is secreted by one of the hybridomas of reference numbers CNCM I-4479, CNCM I-4480 and CNCM I-448, as defined above.

Still another aim of the invention is to provide a composition as defined above, for its use as a prognostic, diagnostic or monitoring tool for assessing a grafted organ distress, notably of graft or transplant rejection.

The terms "prognostic" and "diagnostic" relate to respectively determining if a graft or transplant rejection is to occur or is occurring in a mammal.

By the term "grafted organ distress" is meant grafted organ dysfunction or grafted organ failure. Loss of organ function may results for example, but is not restricted to, accumulation of substances normally eliminated by the organ or inability for the organ to perform its physiological anabolic or catabolic function. Organ failure may be related to, but is not restricted to, necrosis, fibrosis or inflammatory process.

For example, renal distress can be associated with several damages of grafted kidney such as acute tubular necrosis (ATN), chronic interstitial tubular necrosis (CITN), post transplant lymphoproliferative disease (PTLD), extracapillary glomerulonecrosis or graft rejection which is the highest grade of renal distress.

Grafted Organ distress is not restricted to grafted kidney but might also occur in other grafted or transplanted organ and tissues such as lung, heart, liver and bone marrow.

By the term "graft" is meant introduction of cells or tissue in the body of a recipient mammal that come from another individual of the same species (allograft) or from a different species (xenograft) in order to restore a function altered by a disease or a damage. Hematopoietic cells, cord blood cells, pancreatic beta-cells cells illustrate the most commonly performed cells graft. Skin, bone marrow, part of the liver or corneal grafts are given as examples of tissue graft.

By the term "transplant" is meant introduction of an organ removed from the body of a donor mammal to the body of a recipient mammal whose organ can not exert correctly its function anymore. When organ donor and organ recipient mammals are from the same species it is called allotransplantation. Xenotransplantation occurs when the organ donor and the organ recipient are from two different species. The most currently transplanted organs in mammals are lung, kidney, heart, liver, pancreas and intestine. All the previously mentioned types of graft or transplantation are given to illustrate the field of the invention but should not be used to restrain the scope of the present invention.

By the expression "graft or transplant rejection" is meant that partial or total destruction of grafted cells, tissue or organ are destroyed by the immune system of the recipient. Three type of organ rejection have been identified: hyperacute, acute and chronic. Hyperacute rejection generally occurs within twenty four hours of the transplantation. Acute rejection occurs within the first six months of the transplantation whereas chronic rejection could appear between six months to several years after transplantation despite the use of immunosuppressive drugs.

The present invention also provides a method for predicting or performing the *in vitro* prognosis or diagnosis of a grafted organ distress, notably of graft or transplant rejection in a mammal comprising the following steps:
(i) determining the presence or amount of peptide of SEQ ID NO: 1,
in a biological sample of said mammal.

In another embodiment, the present invention provides a method for predicting or performing the *in vitro* prognosis or diagnosis a grafted organ distress, notably of graft or transplant rejection in a mammal comprising the following steps:
(i) determining the presence or amount of antibodies raised against peptide of SEQ ID NO:1,
in a biological sample of said mammal.

The present disclosure also describes a method for predicting or performing the *in vitro* prognosis or diagnosis of a grafted organ distress, notably of graft or transplant rejection in a mammal comprising the following steps:
(i) determining the presence or amount of fragments of peptide of SEQ ID NO: 1,
in a biological sample of said mammal.

In another embodiment, the present disclosure also describes a method for predicting or performing the *in vitro* prognosis or diagnosis a grafted organ distress, notably of graft or transplant rejection in a mammal comprising the following steps:
(i) determining the presence or amount of antibodies raised against fragments of peptide of SEQ ID NO:1,
in a biological sample of said mammal.

The term "mammal" includes human as well as other mammals such as e.g pigs, cows, horses, rabbits, cats, dogs. The mammal can be a female or a male, an adult or a child.

According to the invention, detecting or determining the level of peptide of SEQ ID NO: 1, or of antibodies raised against peptide of SEQ ID NO: 1 may be performed by any techniques known in the art and preferably by using an immunoassay method.

According to the present disclosure, detecting or determining the level of fragments of peptide of SEQ ID NO: 1, or of antibodies raised against fragments of peptide of SEQ ID NO: 1 may be performed by any techniques known in the art and preferably by using an immunoassay method.

By the term "immunoassay" is meant any method wherein the level of peptide of SEQ ID NO: 1 or antibody specifically raised against peptide of SEQ ID NO: 1 peptide is determined using at least one peptide and/or one antibody as defined above in the specification. Immunoassay methods are well known to one of ordinary skill in the art and can have various formats for example in solid or liquid phase, in one or two steps, by a sandwich or a competitive method.

As for example, the colloidal gold immuno-chromatographic assay (CIA) may be used **(**Cao et al., Am. J. Trop. Med. Hyg. 2007, 76(3), 553-558). This type of immunoassay is particularly easy to implement and qualitative results are obtained in less than 30 minutes, and preferably in less than 20 minutes and more preferably in 10 minutes.

The presence of the antigen in the biological sample may be revealed by a gold colloidal labelled detection antibody, said antibody is able to bind to the peptide or protein of interest, by recognising an epitopic site which is different from that recognised by the capture antibody. Preferably, the capture antibody is bound to solid support, in particular cellulose acetate membrane, and the gold colloidal labelled antibody is considered as the migrating antibody.

As for other examples, ELISA assays, radio-immunoassays or any other detection method may be used to reveal the presence of formed antigen-antibody complexes. Thus, different type of labelling of ligands and/or antibody may be envisaged (radioactive, enzymatic, fluorescent, ...).

Measurement determination of peptide of SEQ ID NO: 1 or antibody specifically raised against peptide of SEQ ID NO: 1 peptide is performed on biological sample from a grafted or transplanted recipient a few hours or days after the graft or transplant has occurred. The biological sample can be a body fluid sample or a solid sample such as biopsy of grafted organ or tissue.

By the expression "body fluid sample" is meant any type of sample suitable for use in the methods according to the invention. The biological sample can be selected from the group constituted of a blood sample, a serum sample, a plasma sample, a urine sample or a saliva sample.

The present invention also relates to a kit for the *in vitro* prognosis or diagnosis of a grafted organ distress, notably of graft or transplant rejection comprising:
(i) at least one antibody suitable for binding an epitope comprising or consisting of peptide of SEQ ID NO:1,
(ii) at least one antibody coupled to a detection system suitable for binding the antibody-antigen complex,
and optionally at least one peptide of SEQ ID NO: 1.

The present disclosure also describes a kit for the *in vitro* prognosis or diagnosis of a grafted organ distress, notably of graft or transplant rejection comprising:
(i) at least one antibody suitable for binding an epitope comprising or consisting of fragments of peptide of SEQ ID NO: 1,
(ii) at least one antibody coupled to a detection system suitable for binding the antibody-antigen complex,
and optionally at least one fragment of peptide of SEQ ID NO: 1.

The present invention also relates to a kit for *in vitro* prognosis or diagnosis of a grafted organ distress, notably of graft or transplant rejection comprising:
(i) one antibody, that binds specifically to an epitope consisting of the peptide of the amino acid sequence SEQ ID NO:1 or that is secreted by one of the hybridomas of reference numbers CNCM I-4479, CNCM I-4480 and CNCM I-448, as defined above, suitable for binding an epitope consisting of peptide consisting of the amino acid sequence SEQ ID NO:1,
(ii) one antibody coupled to a detection system suitable for binding the antibody-antigen complex,
   and optionally one peptide consisting of the amino acid sequence SEQ ID NO: 1.

The present invention also relates to the use of peptide of SEQ ID NO: 1 or of fragments of said peptide as a marker for the *in vitro* prognosis or diagnosis of a grafted organ distress, notably of graft or transplant rejection in a mammal.

The present invention also relates to the use of a nucleic acid molecule of SEQ ID NO: 2 capable of encoding a peptide of SEQ ID NO: 1 as a marker for the *in vitro* prognosis or diagnosis of a grafted organ distress, notably of graft or transplant rejection in a mammal.

By the term "marker" is also meant "biomarker", it relates to any protein and/or nucleic acid molecule which expression modulation is useful for prognosing or diagnosing a graft or transplant rejection.

The following examples 1 to 4 and figures 1 to 2 illustrate the invention.
Figure 1 presents tissular localisation of protein from which VEA195 has been isolated. Renal biopsies were performed on recipients suffering or not from graft rejection according to example 2:
   A - Grafted patient not suffering from rejection
   B - Grafted patient suffering from rejection
Figure 2 presents colloïdal gold immuno-chromatographic assay (CIA) results. Strips for diagnosis of whole protein from which VEA195 peptide derived in sera of organ recipient according to example 3:
   (a) - Grafted patient not suffering from rejection
   (b) - Grafted patient suffering from rejection

### Example 1: Isolation, extraction, purification of the 25-30 kDa protein and synthesis of the VEA195 peptide corresponding to the endogenous immunogenic peptide identified on the 25-30 kDa protein.

### 1.1- Protocol

Five sera samples were collected form renal grafted patient and were cleared of human immunoglobulin (IgG) by protein G sepharose 4 fast flow (Pharmacia, Uppsala, Sweden). A total volume of 15 ml was obtained and loaded onto a Sephadex G-75 (Pharmacia, Uppsala, Sweden) chromatography column for separation by using chloroform methanol 3/1 buffer. Eluates of 200 µl fractions were collected and the analysis of fractions n°4-5 (400 µl) showed the presence of the 25-30 kDa protein.

The 400 µl were submitted at a dialysis protocol in tubing (Sigma-Aldrich, France) by submersion in 4 L of PBS overnight at 4°C. A final volume of 200 µl of dialysate was finally collected and allowed the identification of a small sequence of 1.2 kDa that has immunogenic properties. The sequencing of this fraction has been defined as VEA195. This peptide has then been synthesized.

VEA195 peptide was assembled on an automated synthesizer by well known method for a person skilled in the art. Briefly, solid phase peptide synthesis was performed using a Boc/Benzyl strategy. Each aminoacid was coupled with a five fold excess, each coupling was performed in DMF in presence of PyBPO, HObt and DIEA. The peptide was cleaved from the resin by treatment with hydrogen fluoride and purified by reverse phase chromatography. The fractions with purity higher than 95% were pooled.

### 1.2 - Results

VEA195 peptide was controlled for identity by analytical mass spectrometry and purity by analytical HPLC using a nucleosil C18 250x4.6mm column. A 10 to 60% gradient of acetonitrile was used for this purification. The electric potential difference of the collected fraction was measured in order to obtain a chromatographic profile. The only peak on the chromatographic profile corresponds to VEA195 peptide that was dried, resuspended in purified water and then used for immunization of mouse in order to produce specific antibodies.

### Example 2: Immunohistochemistry on renal tissues

### 2.1 - Protocol

Five-micron sections of formalin-fixed, paraffin-embedded grafted kidney biopsies from grafted patients were cut and mounted on slides. Immunohistochemical (IHC) determination of VEA195 previously sequenced from 25-30 kDa protein was performed on an automated staining system (Ventana Medical Systems Tucson, Arizona USA) according to the manufacturer's recommendations. These slides were deparaffined in xylene, dehydrated through three alcohol passages and transferred to Ventana Kit including solution blocking endogenous peroxidase activity. Anti-VEA195 mouse monoclonal antibodies as 2C7D10E5 (E5), 4H7A7G9 (G9) and 1H8C9C10C7 (C7) were also inserted on the platform and automatically distributed in dilution at 1:100 during the assay. These slides were developed in DAB Map (DAB = di-amino-benzidine) and counterstained with hematoxylin (Ventana) and finally mounted in toluene solution (Eukit, CML, Nemours, France).

### 2.2 - Results

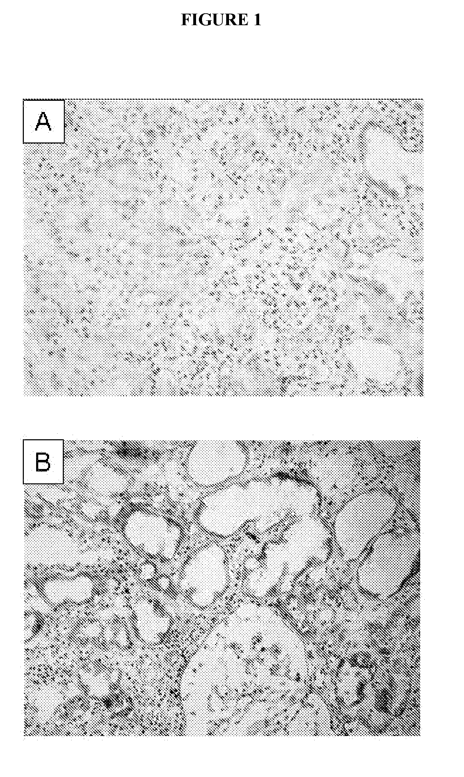

They are illustrated in figure 1. Biopsy from renal grafted recipient suffering from graft rejection shows a typical 25-30 kDa protein staining both on basal glomerular membrane and on tubules part of the kidney. The cytoplasmic staining in tubular cells is very intense (figure 1-B). No 25-30 kDa protein staining appears in the biopsy from healthy (i.e not undergoing graft rejection) renal grafted recipient (figure 1-A).

### Example 3: Immuno-ascendant chromatography results

### 3.1 - Monoclonal antibodies synthesis

About 50 µg of VEA195 peptide diluted in PBS was injected per mouse to a total of 4 mice. The first injection was done intraperitoneally using VEA195 peptide emulsified in complete Freund adjuvant. New injection was performed at day 21 and 42 with test bleeding at day 0, 14, 35 and 56. Upon completion of the last test bleeding an ELISA test was performed. After the last bleed the spleen of the best reacting mouse was operatively taken off. Lymphocytes were isolated and taken up to culture medium.

To keep immunoglobulin-producing lymphocytes alive they were fused with a cell line of mouse myeloma cell line [myeloma SP2/0-Ag14 (ATCC CRL 8287)] in presence of polyethylenglycol. A separation of fused and unfused lymphocytes and myeloma cells was performed on plates filled with HAT medium (Hypoxanthin-Aminopterin-Thymidin). The surviving cells are collected and constitute the hybridoma. The supernatants of the cell cultures are tested on the presence of antigen-specific antibodies and hybridoma cells are frozen to be further grown. Isolation of really monoclonal antibody-producing hybridoma cell line is then performed by diluting the mixture in multiwell plates. By this process one "mother cell" is selected secreting one unique antibody after a cascade of new cell's selection and cloning, ELISA and microscopic controls.

By using this technique, IgG1 or IgG2 kappa isotype monoclonal antibodies were raised against VEA195 peptide: 2C7D10E5 (E5), 4H7A7G9 (G9) and 1H8C9C10C7 (C7).

### 3.2 - Immuno assay protocol

The double-antibody sandwich technology is used in this assay to enable the detection of whole protein from which VEA195 peptide derived in sera of grafted patients. This test is based on immunochromatography using two specific antibodies raised against VEA195 peptide and a non-specific antibody raised against mouse Ig. The monoclonal E5 capture antibody specifically recognises an epitope of VEA195. Colloidal gold-conjugated G9 migrant antibody recognises a second epitope of VEA195 peptide. Anti-mouse Ig is a control capture antibody that serves as a negative control. The three antibodies were bound linearly to cellulose acetate membrane. The strip is immerged in an assay tube containing the diluted serum of patient in migration buffer (Na₂HPO₄ 50 mM, BSA 1% and NaN₃ 0,10% pH 7.4 from BIOSYNEX). The complex composed of E5 capture antibody-protein from which VEA195 peptide derived-colloidal gold-conjugated G9 migrant antibody forms the test line and the complex composed of Anti-mouse Ig capture antibody- colloidal gold-conjugated G9 migrant antibody forms the control line. The migration of colloidal gold-conjugated antibodies occurs along the test strip. The result was read after 10 minutes. When both the test line and the control line are red, it means that the corresponding sera sample was positive. If only the control line is red, a negative result is indicated.

### 3.3 - Results

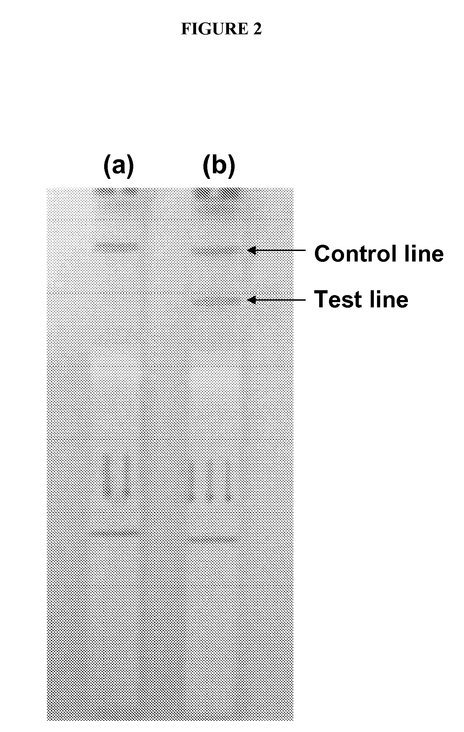

They are illustrated in figure 2. Two lines are present on the strip performed with serum of renal grafted patient suffering from graft rejection (figure 2-b): the upper line corresponds to the control line and the lower band is the test line. Only the control line can be seen is the strip test performed with the serum of renal grafted patient not suffering from graft rejection (figure 1-a).

A comparative study between colloidal gold immuno-chromatographic assay (CIA) and immunohistochemistry (IHC), performed by hematoxylin and eosin stained section of the grafted organ was done on a total of 20 serum samples of renal grafted patients (table 1, below). Final diagnosis was established according to IHC criteria. All experiments were performed with the samples coded and blinded. Results are gathered in Table 1.

**Table 1: Comparative study between CIA and ICH. AHR = acute humoral rejection; ACR = acute cellular rejection; PTLD = post transplant lymphoproliferative disease; ATN = acute tubular necrosis; BP = borderline pathology; CITN = chronic interstitial tubular necrosis; ND = not done.**

| **Patient categories** | **Patient ID** | **Date of sampling** | **IHC analysis** | **CIA analysis** | **Diagnosis defined by pathology** |
|---|---|---|---|---|---|
| A1 | 16 | 22/04/2008 | - | - | no rejection |
| | 17 | 27/05/2008 | - | - | |
| | 27 | 26/06/2008 | - | - | |
| A2 | 11 | 05/02/2008 | - | - | acute pyelonephritis - no rejection |
| | 13 | 07/02/2008 | - | - | |
| B | 1 | 25/10/2006 | BP | + | AHR |
| | | 13/02/2008 | + | + | |
| | | 27/06/2008 | + | + | |
| | 12 | 18/06/2008 | + | + | |
| C | 14 | 25/06/2008 | BP | + | ACR |
| | 28 | 11/06/2008 | - | + | ND |
| | | 18/08/2008 | + | + | ACR |
| | 34 | 28/02/2006 | BP | ND | ND |
| | | 08/08/2006 | BP | ND | ND |
| | | 28/01/2008 | BP | + | ACR + PTLD |
| | 15 | 02/05/2007 | + | + | ACR + cortical necrosis |
| D | 9 | 31/01/2008 | + | + | ATN |
| | 10 | 23/01/2008 | + | + | CITN |
| | 29 | 29/01/2008 | - | + | cryoglobulinemia |
| | 30 | 09/04/2008 | + | + | extracapillary glomerulonecrosis |

The 5 serum samples that were found negative with both assays belong to the grafted patient not suffering from graft rejection. This group of patient can be subdivided in two categories: A1 are healthy recipients and A2 are recipients suffering from bacterial infection. Patients belonging to these A1 and A2 categories do not suffer from any renal distress. 13 sera were positive for CIA using anti-VEA195 antibodies. Among those positive samples, 4 were related to acute humoral rejection (AHR) and 4 to acute cellular rejection (ACR). Other forms of renal distress were reactive in 4 serum samples, mainly related to acute tubular necrosis (ATN), chronic interstitial tubular necrosis (CITN) or extracapillary glomerulonecrosis. Patients 1 (first sample), 14 and 34 (third sample) were found borderline pathology (BP, i.e inflammatory molecules were found in limited quantity) with IHC requiring performing another biopsy. The biopsies performed a few months later confirmed the results obtained previously with the CIA assay. These results clearly demonstrated that anti-VEA195 antibodies are reliable biomarkers for renal distress including graft rejection.

### Example 4: ELISA assays

### 4.1 - Protocol

Microtiter plates are coated with 100 µl of sera from patients diluted in 20 µl of carbonate buffer 50 mM, pH 9.4 overnight at 4°C. Microtiter plates are washed up 3 times with PBS-0.05% Tween 20 buffer. The plates were incubated with 250 µl of PBS buffer and 150 µl of a solution at 5% BSA at room temperature for 2 hours and washed up 3 times with PBS-0.05% Tween 20 buffer. Each well received 100 µl of a 1:4000 dilution E5 antibody in PBS-BSA 1% and the plates are incubated at room temperature for 1 hour. Microtiter plates are washed up 3 times with PBS-0.05% Tween 20 buffer. Peroxydase conjugated goat anti-mouse IgG antibody is added at 1:3000 to each well and incubated for 30 minutes at 37°C. Microtiter plates are washed up 3 times with PBS-0.05% Tween 20 buffer. OPD (Ortho-Phenylenediamine) substrate is then added for 10 minutes. Reaction is stopped by addition of a 4 mM solution of H₂SO₄. Microtiter plates are then read at 415 nm by an optical lecturer.

### 4.2 - Results

Results are shown below on table 2. ELISA assay was done with serum samples of blood donors (T) to determine the cut-off value which appears to be upper than 0.061 OD unit in this assay. Patients are tested for graft rejection several months after the ELISA assays.

**Table 2: anti-VEA195 ELISA assay performance on grafted recipient serum samples. AHR = acute humoral rejection; ACR = acute cellular rejection**

| **Patient categories** | **Patient ID** | **Date of sampling** | **OD values (415 nm)** | **Diagnosis defined by pathology** |
|---|---|---|---|---|
| Blood donors | 100 | 01/03/2007 | 0,048 | None |
| | | 01/04/2007 | 0,051 | |
| | | 08/05/2007 | 0,045 | |
| | | 15/05/2007 | 0,050 | |
| | 200 | 20/07/2007 | 0,039 | |
| | | 03/08/2007 | 0,054 | |
| | | 18/08/2007 | 0,061 | |
| | | 30/08/2007 | 0,057 | |
| | | 12/09/2007 | 0,048 | |
| | 300 | 12/04/2008 | 0,048 | |
| | | 30/04/2008 | 0,044 | |
| | | 15/05/2008 | 0,038 | |
| | | 27/05/2008 | 0,047 | |
| A1 | 400 | 10/07/2008 | 0,047 | no rejection |
| | 500 | 15/08/2008 | 0,061 | |
| B | 600 | 20/04/2006 | 0,093 | AHR |
| | | 12/05/2006 | 0,044 | |
| | | 30/05/2006 | 0,056 | |
| | 700 | 30/04/2007 | 0,055 | |
| | | 12/05/2007 | 0,057 | |
| | | 30/05/2007 | 0,084 | |
| | | 24/06/2007 | 0,137 | |
| | | 15/08/2007 | 0,041 | |
| C | 800 | 05/05/2007 | 0,288 | ACR |
| | | 23/06/2007 | 0,135 | |
| | 900 | 08/02/2006 | 2,436 | |
| | | 22/02/2006 | 2,691 | |
| | | 15/03/2006 | 1,281 | |
| | 1000 | 30/03/2005 | 2,377 | |
| | | 06/04/2005 | 2,638 | |
| | | 18/04/2005 | 2,560 | |
| | | 30/04/2005 | 2,557 | |
| | | 08/05/2005 | 2,797 | |
| | 1100 | 03/04/2006 | 0,157 | |
| | | 15/05/2006 | 0,161 | |
| | | 28/05/2006 | 2,661 | |

34 serum samples from 8 renal grafted recipients were assayed with anti-VEA195 E5 antibody. In blood donors, i.e people who are not grafted, the OD value is below 0.061. In grafted patients who do not suffer from graft rejection several months after ELISA assays were performed, the OD value stand also below 0.061. For grafted patients undergoing graft rejection several months after ELISA assay were performed, the OD value rise from 0.084 to more than 2.7. Consequently, the amount of the 25-30 kDa protein, from which VEA195 peptide derived, in blood sample could be a good tool for prediction of graft rejection. The kinetic expression profile of the 25-30 kDa protein in renal transplant recipients is probably influenced by the status of the disease and related to the immunosuppressive treatment of patients.

The assays show that the present invention could be an efficient tool and could provide methods to evaluate grafted organ distress and notably the most deleterious stage illustrated here by graft rejection.

## Claims

1. A peptide consisting of the amino acid sequence SEQ ID NO: 1.

2. A nucleic acid molecule capable of encoding a peptide according to claim 1 wherein said nucleic acid is SEQ ID NO: 2.

3. An antibody or fragment thereof that binds specifically to an epitope consisting of the peptide according to claim 1.

4. Hybridomas deposited at the CNCM (Paris) under the Budapest Treaty on May 4, 2011, under reference numbers CNCM I-4479, CNCM I-4480 and CNCM I-448, that secretes an antibody or fragment thereof that binds specifically to an epitope consisting of the peptide according to claim 1.

5. An antibody or fragment thereof according to claim 3 that is secreted by one of the Hybridomas of reference numbers CNCM I-4479, CNCM I-4480 and CNCM I-4481, as defined in claim 4.

6. A composition comprising a peptide according to claim 1 or an antibody according to claim 3 or 5.

7. Composition according to claim 6 for its use as a prognostic, diagnostic or monitoring tool for assessing a grafted organ distress, notably of graft or transplant rejection.

8. An *in vitro* method for predicting or performing the prognosis or diagnosis of a grafted organ distress, notably of graft or transplant rejection in a mammal comprising the following steps :
(i) determining the presence or amount of peptide consisting of the amino acid sequence SEQ ID NO: 1,
in a biological sample of said mammal.

9. An *in vitro* method for predicting or performing the prognosis or diagnosis of a grafted organ distress, notably of graft or transplant rejection in a mammal comprising the following steps :
(i) determining the presence or amount of antibodies raised against peptide of SEQ ID NO:1,
in a biological sample of said mammal.

10. The method according to any one of claim 8 or 9, wherein the level of peptide consisting of the amino acid sequence SEQ ID NO: 1, or of antibodies raised against peptide of SEQ ID NO: 1 is measured using an immunoassay.

11. The method according to any one of claims 8 to 10, wherein the antibody is secreted by one of the hydridomas deposited at the CNCM (Paris) under the Budapest Treaty on May 4, 2011, under references numbers CNCM I-4479, CNCM I-4480 and CNCM I-4481.

12. The method according to claim 8 or 9, wherein the biological sample is a body fluid sample.

13. A kit for *in vitro* prognosis or diagnosis of a grafted organ distress, notably of graft or transplant rejection comprising :
(i) one antibody according to claim 3 or 5 suitable for binding an epitope consisting of peptide consisting of the amino acid sequence SEQ ID NO: 1,
(ii) one antibody coupled to a detection system suitable for binding the antibody-antigen complex,
and optionally one peptide consisting of the amino acid sequence SEQ ID NO: 1.

14. The *in vitro* use of peptide consisting of the amino acid sequence SEQ ID NO: 1 as a marker for the prognosis or diagnosis of a grafted organ distress, notably of graft or transplant rejection in a mammal.

15. The *in vitro* use of a nucleic acid molecule, wherein said nucleic acid is SEQ ID NO: 2, capable of encoding a peptide of SEQ ID NO: 1 as a marker for the prognosis or diagnosis of a grafted organ distress, notably of graft or transplant rejection in a mammal.

## Patentansprüche

1. Peptid bestehend aus der Aminosäuresequenz SEQ ID NR. 1.

2. Nukleinsäuremolekül, das in der Lage ist, ein Peptid nach Anspruch 1 zu codieren, wobei es sich bei der Nukleinsäure um SEQ ID NR. 2 handelt.

3. Antikörper oder ein Fragment davon, der bzw. das spezifisch an ein aus dem Peptid nach Anspruch 1 bestehendes Epitop bindet.

4. Hydridome, die im Einklang mit dem Budapester Vertrag vom 4. Mai 2011 unter den Bezugsnummern CNCM 1-4479, CNCM 1-4480 und CNCM 1-448 beim CNCM (Paris) hinterlegt sind, die einen Antikörper oder ein Fragment davon sezernieren, der bzw. das spezifisch an ein aus dem Peptid nach Anspruch 1 bestehendes Epitop bindet.

5. Antikörper oder ein Fragment davon nach Anspruch 3, der bzw. das von einem der Hybridome mit der Bezugsnummer CNCM 1-4479, CNCM 1-4480 und CNCM 1-4481 sezerniert wird, nach der Definition in Anspruch 4.

6. Zusammensetzung, umfassend ein Peptid nach Anspruch 1 oder einen Antikörper nach Anspruch 3 oder 5.

7. Zusammensetzung nach Anspruch 6 zur Verwendung als prognostisches Hilfsmittel, diagnostisches Hilfsmittel oder Überwachungshilfsmittel zur Beurteilung einer Überlastung eines transplantierten Organs, d. h. einer Graft- bzw. Transplantatabstoßung.

8. In-vitro-Verfahren zum Prognostizieren oder Erstellen der Prognose oder Diagnose der Überlastung eines transplantierten Organs, d. h. einer Graft- bzw. Transplantatabstoßung, in einem Säugetier, welches die folgenden Schritte umfasst:
(i) Bestimmen des Vorhandenseins oder der Menge eines Peptids, das aus der Aminosäuresequenz SEQ ID NR. 1 besteht,
in einer biologischen Probe des Säugetiers.

9. In-vitro-Verfahren zum Prognostizieren oder Erstellen der Prognose oder Diagnose der Überlastung eines transplantierten Organs, d. h. einer Graft- bzw. Transplantatabstoßung, in einem Säugetier, welches die folgenden Schritte umfasst:
(i) Bestimmen des Vorhandenseins oder der Menge von Antikörpern, die gegen ein Peptid mit SEQ ID NR. 1 erzeugt worden sind,
in einer biologischen Probe des Säugetiers.

10. Verfahren nach einem der Ansprüche 8 oder 9, wobei die Menge des aus der Aminosäuresequenz SEQ ID NR. 1 bestehenden Peptids bzw. der gegen ein Peptid mit SEQ ID NR. 1 erzeugten Antikörper mithilfe eines Immunassays gemessen wird.

11. Verfahren nach einem der Ansprüche 8 bis 10, wobei der Antikörper von einem der Hybridome sezerniert wird, die im Einklang mit dem Budapester Vertrag vom 4. Mai 2011 unter den Bezugsnummern CNCM 1-4479, CNCM I-4480 und CNCM 1-4481 beim CNCM (Paris) hinterlegt sind.

12. Verfahren nach Anspruch 8 oder 9, wobei es sich bei der biologischen Probe um eine Körperflüssigkeitsprobe handelt.

13. Kit für die In-vitro-Prognose oder -Diagnose der Überlastung eines transplantierten Organs, d. h. einer Graft- bzw. Transplantatabstoßung, umfassend:
(i) einen Antikörper nach Anspruch 3 oder 5, der zum Binden an ein Epitop bestehend aus einem aus der Aminosäuresequenz SEQ ID NR. 1 bestehenden Peptid geeignet ist,
(ii) einen Antikörper, der an ein Detektionssystem gekuppelt ist, das zum Binden des Antikörper-Antigen-Komplexes geeignet ist,
und wahlweise einem Peptid, das aus der Aminosäuresequenz SEQ ID NR. 1 besteht.

14. In-vitro-Verwendung eines Peptids, das aus der Aminosäuresequenz SEQ ID NR. 1 besteht, als Marker für die Prognose oder Diagnose der Überlastung eines transplantierten Organs, d. h. einer Graft- bzw. Transplantatabstoßung, bei einem Säugetier.

15. In-vitro-Verwendung eines Nukleinsäuremoleküls, wobei es sich bei der Nukleinsäure um SEQ ID NR. 2 handelt, die zum Codieren eines Peptids mit SEQ ID NR. 1 in der Lage ist, als Marker für die Prognose oder Diagnose der Überlastung eines transplantierten Organs, d. h. einer Graft- bzw. Transplantatabstoßung, bei einem Säugetier.

## Revendications

1. Peptide consistant en la séquence d'acides aminés SED ID NO : 1.

2. Molécule d'acides nucléiques capable de coder un peptide selon la revendication 1 dans laquelle ledit acide nucléique est SEQ ID NO :2.

3. Anticorps ou un fragment de celui-ci qui se lie spécifiquement à un épitope consistant en le peptide selon la revendication 1.

4. Hybridomes déposés à la CNCM (Paris) selon le Traité de Budapest du 4 Mai 2011, sous les numéros de référence CNCM I-4479, CNCM I-4480 et CNCM I-4481, qui sécrètent un anticorps ou un fragment de celui-ci qui se lie spécifiquement à un épitope consistant en le peptide selon la revendication 1.

5. Anticorps ou un fragment de celui-ci selon la revendication 3 qui est sécrété par l'un des hybridomes de numéros de référence CNCM I-4479, CNCM I-4480 et CNCM I-4481, tels que définis à la revendication 4.

6. Composition comprenant un peptide selon la revendication 1 ou un anticorps selon la revendication 3 ou 5.

7. Composition selon la revendication 6 pour son utilisation dans le pronostic, le diagnostic ou comme outil de suivi pour l'évaluation d'un problème avec un organe greffé, notamment le rejet d'une greffe ou d'un transplant.

8. Méthode *in vitro* pour prédire et réaliser le pronostic ou le diagnostic d'un problème avec un organe greffé, notamment le rejet d'une greffe ou d'un transplant chez un mammifère comprenant les étapes suivantes :
(i) la détermination de la présence ou d'une quantité de peptide consistant en la séquence d'acides aminés SEQ ID NO :1,
dans un échantillon biologique dudit mammifère.

9. Méthode *in vitro* pour prédire ou réaliser le pronostic ou le diagnostic d'un problème avec un organe greffé, notamment le rejet d'une greffe ou d'un transplant chez un mammifère. comprenant les étapes suivantes :
(i) la détermination de la présence ou d'une quantité d'anticorps dirigés contre le peptide de SEQ ID NO :1,
dans un échantillon biologique dudit mammifère.

10. Méthode selon l'une quelconque des revendications 8 ou 9, dans laquelle le niveau de peptide consistant en la séquence d'acides aminés SEQ ID NO :1, ou d'anticorps dirigés contre le peptide de SEQ ID NO :1 est mesuré en utilisant un test immunologique.

11. Méthode selon l'une quelconque des revendications 8 à 10, dans laquelle l'anticorps est sécrété par l'un des hybridomes déposés à la CNCM (Paris) selon le Traité de Budapest du 4 Mai 2011, sous les numéros de référence CNCM I-4479, CNCM I-4480 et CNCM I-4481.

12. Méthode selon la revendication 8 ou 9, dans laquelle l'échantillon biologique est un échantillon de fluide corporel.

13. Kit pour le pronostic ou le diagnostic *in vitro* d'un problème avec un organe greffé, notamment le rejet d'une greffe ou d'un transplant comprenant :
(i) un anticorps selon la revendication 3 ou 5 capable de se lier à un épitope consistant en le peptide consistant en la séquence d'acides aminés SEQ ID NO :1
(ii) un anticorps couplé à un système de détection capable de se lier au complexe anticorps-antigène,
et optionnellement un peptide consistant en la séquence d'acides aminés SEQ ID NO :1.

14. Utilisation *in vitro* du peptide consistant en la séquence d'acides aminés SEQ ID NO :1 comme marqueur pour le pronostic ou le diagnostic d'un problème avec un organe greffé, notamment le rejet d'une greffe ou d'un transplant chez un mammifère.

15. Utilisation *in vitro* d'une molécule d'acides nucléiques, dans laquelle ledit acide nucléique est SEQ ID NO :2, capable de coder un peptide de SEQ ID NO :1 comme marqueur pour le pronostic ou le diagnostic d'un problème avec un organe greffé, notamment le rejet d'une greffe ou d'un transplant chez un mammifère.


### SEQUENCE LISTING

<110> Félix Agbalika Agbalika, Félix
<120> Biomarkers for in vitro prognosis and diagnosis of graft and transplant rejection
<130> IOB 11 AA AGB VEA1
<160> 2
<170> PatentIn version 3.5
<210> 1
   <211> 11
   <212> PRT
   <213> Artificial Sequence
<220>
   <223> peptide derived from Homo sapiens
<400> 1
   Lys Glu Thr Ile Asn Glu Glu Ala Ala Glu Trp
   1               5                   10
<210> 2
   <211> 33
   <212> DNA
   <213> Artificial Sequence
<220>
   <223> nucleic acide molecule derived from Homo sapiens
<220>
   <221> misc_feature
   <222> (3)..(3)
   <223> R is a or g
<220>
   <221> misc_feature
   <222> (6) .. (6)
   <223> R is a or g
<220>
   <221> misc_feature
   <222> (9) .. (9)
   <223> N is a, c, t or g
<220>
   <221> misc_feature
   <222> (12)..(12)
   <223> H is a, c, or t
<220>
   <221> misc_feature
   <222> (15)..(15)
   <223> Y is c or t
<220>
   <221> misc_feature
   <222> (18)..(18)
   <223> R is a or g
<220>
   <221> misc_feature
   <222> (21) .. (21)
   <223> R is a or g
<220>
   <221> misc_feature
   <222> (24) .. (24)
   <223> N is a, c, t or g
<220>
   <221> misc_feature
   <222> (27) .. (27)
   <223> N is a, c, t or g
<220>
   <221> misc_feature
   <222> (30)..(30)
   <223> R is a or g
<400> 2
   aargaracna thaaygarga rgcngcngar tgg 33